# EUROPÄISCHE PATENTSCHRIFT

(11) **EP 0 924 182 B1**
(45) Veröffentlichungstag und Bekanntmachung des Hinweises auf die Patenterteilung: **02.07.2003**
(21) Anmeldenummer: 98123784.5
(22) Anmeldetag: 15.12.1998
(51) Int. Cl.: C07C 45/50, B01J 31/24, B01J 31/02

(54) **Verfahren zur Herstellung von Aldehyden**
Process for the preparation of aldehydes
Procédé pour la préparation d' aldéhydes

(30) Priorität: 22.12.1997 DE 19756946
(43) Veröffentlichungstag der Anmeldung: 23.06.1999
(73) Patentinhaber: Celanese Chemicals Europe GmbH, 60439 Frankfurt am Main (DE)
(72) Erfinder: Bahrmann, Helmut Dr., 46499 Hamminkeln (DE)

(56) Entgegenhaltungen:
- EP-A- 0 107 430
- EP-A- 0 216 315
- EP-A- 0 776 880
- DE-A- 19 532 393
- US-A- 3 931 332
- US-A- 3 976 703
- US-A- 4 716 250

## Beschreibung

Die vorliegende Erfindung betrifft ein Verfahren zur Herstellung von Aldehyden durch Hydroformylierung von Olefinen oder olefinisch ungesättigten Verbindungen in Gegenwart mindestens einer Rhodiumverbindung und in Gegenwart einer nichtwäßrigen ionogenen Ligandflüssigkeit der allgemeinen Formel (Q^{⊕})ₐ A^{a-}, in der Q^{⊕} ein quaternäres einfach geladenes Ammonium- und/oder Phosphonium-Kation ist oder das Äquivalent eines mehrfach geladenen Ammonium- und/oder Phosphonium-Kation ist und A^{a-} ein sulfoniertes Triarylphosphin bedeutet.

Aldehyde besitzen als wertvolle Zwischenprodukte eine große wirtschaftliche Bedeutung. Aus ihnen lassen sich z.B. Alkohole, Carbonsäuren und Amine herstellen, die wiederum als Ausgangsprodukte für die Herstellung wichtiger Endprodukte verwendet werden.

Es ist bekannt, durch Umsetzung von Olefinen mit Kohlenmonoxid und Wasserstoff Aldehyde herzustellen. Die Reaktion wird durch Hydridometallcarbonyle, vorzugsweise solcher der Metalle der Gruppe VIII des Periodensystems der Elemente, katalysiert. Während zunächst Kobalt als Katalysatormetall in großem Umfang technische Anwendung fand, haben sich Verfahren zur Herstellung von Aldehyden, die Rhodium als Katalysatormetall verwenden, in der Zwischenzeit in der Technik etabliert.

Die Herstellung von Aldehyden kann einphasig in einer organischen Phase erfolgen. Dabei liegt der Katalysator, wie z.B. ein Rhodium/Triphenylphosphinkomplex, gelöst in dem organischen Reaktionsgemisch vor. Darüber hinaus kann die Herstellung von Aldehyden auch in Gegenwart eines organischen Lösungsmittels erfolgen. Als Lösungsmittel werden z.B. Toluol, Xylol oder Tetrahydrofuran verwendet.

Ein solches, einphasig durchgeführtes Verfahren ist aus US-A-4,716,250 bekannt. Als Katalysatorsystem verwendet man einen Gruppe VIII Übergangsmetallkomplex und freien Phosphinliganden, wobei es sich bei den Phosphinliganden um ein Ammoniumsalz von monosulfonierten tertiären Arylphosphinen handelt. Die Ammoniumkationen sind so auszuwählen, dass der Übergangsmetallkomplex und der Phosphinligand eine ausreichende Löslichkeit in dem organischen Medium besitzt. Nach erfolgter Hydroformylierung liegt der Katalysator gelöst in dem organischen Medium vor. Durch Destillation wird der gewünschte Aldehyd abgetrennt, während der Katalysator im Destillationsrückstand verbleibt.

Probleme wirft bei diesem Verfahren jedoch die Abtrennung der Reaktionsprodukte und die Wiedergewinnung der im Reaktionsprodukt homogen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde aber nur bei der Hydroformylierung niedriger Olefine, d.h. Olefinen mit bis zu etwa 5 Kohlenstoffatomen im Molekül beschritten werden. Außerdem kann die thermische Belastung des Destillationsgutes zu erheblichen Verlusten an Produkt durch Nebenproduktbildung und Verlusten an Katalysator durch Zersetzung der katalytisch wirksamen Komplexverbindungen führen.

Ein weiteres, einphasig durchgeführtes Hydroformylierungsverfahren ist aus EP-A-0 216 315 bekannt. Auch nach diesem Verfahren verwendet man Ammoniumsalze sulfonierter Triarylphosphine mit ausreichender Löslichkeit in dem organischen Reaktionsmedium. Die Abtrennung des homogen gelösten Katalysators erfolgt durch Zugabe einer wäßrigen Basenlösung, die zu einer Spaltung des Aminsalzes und zur Bildung mit Wasser extrahierfähigen sulfonierten Triarylphosphinen führt. Aus dem wäßrigen katalysatorhaltigen Extrakt wird durch erneute Extraktion mit einem aminhaltigen organischen Lösungsmittel die organische Katalysatorlösung zurückgewonnen.

Auch aus DE-A1-195 32 393 ist ein in homogener Phase durchgeführter Hydroformylierungsprozeß bekannt. Zur Sicherstellung der homogenen Reaktionsführung wird ein polares organisches Lösungsmittel dem Reaktionsgemisch zugesetzt. Als Katalysator verwendet man sulfonierte organische Phsophine mit Ammoniumionen als Gegenionen der Formel NR¹R²R³R^{4⊕}, wobei R¹ bis R⁴ insbesondere Alkylreste mit 1 bis 4 Kohlenstoffatomen bedeuten. Nach erfolgter Hydroformylierungsreaktion wird das polare organische Lösungsmittel von den hochsiedenden Hydroformylierungsprodukten abdestilliert und der Destillationsrückstand mit Wasser extrahiert.

Aus US-A-3,976,703 ist bekannt, daß bei homogenen Hydroformylierungsreaktionen, die unter Kobaltcarbonylkatalyse durchgeführt werden, der Zusatz von sulfonierten Arylphosphinen stabilisierend auf den Katalysatorkomplex wirkt. Als Gegenionen zu den sulfonierten Arylphosphinen verwendet man quaternäre Ammoniumkationen der allgemeinen Formel R'₄N⁺, in denen die Alkylreste 1 bis 20 Kohlenstoffatome enthalten.

Diese Mängel lassen sich vermeiden, wenn die Hydroformylierungsreaktion in einem Zweiphasensystem durchgeführt wird. Ein derartiger Prozeß ist z.B. in der DE-PS 26 27 354 beschrieben. Dieser Prozeß ist charakterisiert durch das Vorliegen einer organischen Phase, die die Ausgangsolefine und das Reaktionsprodukt enthält und einer wäßrigen Phase, in der der Katalysator gelöst ist. Als Katalysatoren werden wasserlösliche Rhodiumkomplexverbindungen eingesetzt, die wasserlösliche Phosphine als Liganden enthalten. Zu den Phosphinen zählen insbesondere Triarylphosphine, Trialkylphosphine und arylierte bzw. alkylierte Diphosphine, deren organische Reste durch Sulfonsäuregruppen oder Carboxylgruppen substituiert sind. Ihre Herstellung ist z.B. aus DE-PS 26 27 354 bekannt.

Der in Gegenwart einer wäßrigen katalysatorhaltigen Phase zweiphasig durchgeführte Hydroformylierungsprozeß bewährt sich besonders bei der Hydroformylierung niedriger Olefine, insbesondere bei Ethylen und Propylen. Setzt man hingegen höhere Olefine wie Hexen, Octen oder Decen ein, so geht der Umsatz deutlich zurück. Verursacht wird der Rückgang des Umsatzes durch die Abnahme der Löslichkeit höherer Olefine in Wasser, da man annimmt, daß die Reaktion zwischen den Reaktanden in der wäßrigen Phase abläuft. Der Olefinumsatz wird aber deutlich erhöht, wenn man der wäßrigen Katalysatorlösung ein Phasentransferreagenz (Lösungsvermittler) zusetzt. Bewährt haben sich nach EP-B-0 562 451 insbesondere kationische Lösungsvermittler der allgemeinen Formel [A-N(R¹R²R³)]⁺E⁻, in der A für einen geradkettigen oder verzweigten Alkylrest mit 6 bis 25 Kohlenstoffatomen steht, R¹, R², R³ gleich oder verschieden sind und geradkettige oder verzweigte Alkylreste mit 1 bis 4 Kohlenstoffatomen bedeuten und E⁻ für ein Anion, insbesondere Sulfat, Tetrafluoroborat, Acetat, Methosulfat, Benzolsulfonat, Alkylbenzolsulfonat, Toluolsulfonat, Lactat oder Citrat steht.

Neben der ausreichenden Löslichkeit des Olefins in der wäßrigen Phase ist für die zweiphasige Durchführung des Hydroformylierungsprozesses in Gegenwart einer wäßrigen katalysatorhaltigen Phase eine ausreichenden Stabilität des umzusetzenden Olefins gegenüber Wasser notwendig. Wasserempfindliche Olefine, wie z.B. Acrylsäureester oder ungesättigte Acetale, lassen sich daher nach diesem Verfahren nicht erfolgreich einsetzen.

Um diesen Nachteil zu überwinden, ohne auf den Vorteil des zweiphasig durchgeführten Hydroformylierungsprozesses zu verzichten, wird die Verwendung von unpolaren Perfluorkohlenwasserstoffen, z.B. von Perfluormethylcyclohexan als nichtwäßrige, nicht mit dem organischen Reaktionsprodukt mischbaren Phase für die katalytische Hydroformylierung von Olefinen vorgeschlagen. Jedoch sind zum Lösen der Rhodiumkomplexe in den Perfluorkohlenwasserstoffen spezielle fluorierte Liganden, wie zum Beispiel Tris-(1H,1H,2H-perfluoroctyl)phosphan notwendig (Science 1994,266,72).

Eine andere Arbeitsweise, katalytische Reaktionen in einem nichtwäßrigen Zweiphasensystem durchzuführen, wird in CHEMTECH, September 1995, Seiten 26 bis 30 beschrieben. Hiernach lassen sich nichtwäßrige ionogene Flüssigkeiten, die schon bei Raumtemperatur flüssig sind, z.B. eine Mischung aus 1,3-Dialkylimidazolium Chlorid, bevorzugt 1-n-Butyl-3-methylimidazolium Chlorid, und Aluminiumchlorid und/oder Ethylaluminiumdichlorid, als nichtwäßrige Lösungsmittel verwenden, die den Katalysatorkomplex gelöst enthalten. Das 1-n-Butyl-3-methylimidazolium Kation wird im Stand der Technik mit BMI^{⊕} abgekürzt. Als Beispiel für derart erfolgreich durchgeführte Reaktionen ist die Olefindimerisierung in Gegenwart von Nickelkomplexen, z.B. die Propendimerisierung zu isomeren Hexenen oder die Butendimerisierung zu Iso-Octenen zu nennen. Das Reaktionsprodukt bildet die obere Phase, während die katalysatorhaltige nichtwäßrige ionogene Flüssigkeit die untere Phase bildet und durch einfache Phasentrennung abgetrennt werden kann. Die katalysatorhaltige nichtwäßrige ionogene Flüssigkeit kann erneut in den Prozeß eingeführt werden.

Aus Am. Chem. Soc., Div. Pet. Chem. 1992, 37, Seiten 780 bis 785 ist bekannt, daß eine nichtwäßrige ionogene Flüssigkeit, bestehend aus l-Butyl-3-methylimidazoliumchlorid und Aluminiumchlorid als Lösungsmittel dient, in dem nach Zusatz von Ethylaluminiumdichlorid und NiCl₂(PR₃)₂ mit R gleich Isopropyl die Dimerisierung von Propen erfolgt.

Die Verwendung von niedrig schmelzenden Phosphoniumsalzen, z.B. von Tetrabutylphosphonium-Bromid als Lösungsmittel in Hydroformylierungsreaktionen wird im Journal of Molecular Catalysis, 47 (1988) Seiten 99 - 115 offenbart. Danach führt die Hydroformylierung von Olefinen, z.B.Octen-1, mit Rutheniumcarbonylkomplexen in Gegenwart von stickstoff- oder phosphorhaltigen Liganden, z.B. von 2,2'-Dipyridin oder 1,2-Bis(diphenylphosphino)ethan, bei Temperaturen von 120 bis 180 °C zu einem Gemisch aus n-Nonanol und n-Nonanal. Bei diesem Verfahren fällt n-Nonanol mit bis zu 69 Gew.-%, bezogen auf das Reaktionsgemisch, an, so daß zur Isolierung des gewünschten n-Nonanal eine aufwendige Destillation erforderlich ist.

Die europäische Patentanmeldung EP-A-0 776 880 offenbart die Hydroformylierung von Olefinen in Gegenwart von quaternären Ammonium- und/oder Phosphoniumsalzen als Lösungsmittel, wobei als Kation vorzugsweise das 1-n-Butyl-3-methylimidazolium Kation 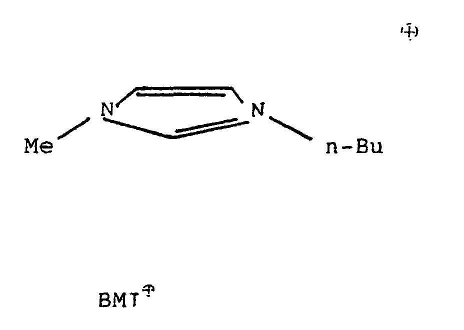 verwendet wird. Aber auch Salze von quaternären Diaminen, bei denen das Kation die allgemeine Formel

R¹R²N^{⊕}=CR³-R⁵-R³C=N^{⊕}R¹R²

hat, wobei R¹, R², R³ gleich oder verschieden sind und Wasserstoff oder einen Kohlenwasserstoffrest mit 1 bis 12 Kohlenstoffatomen und R⁵ Alkylen, z.B. Methylen, Ethylen oder Propylen oder Phenylen, bedeuten, werden verwendet. Geeignete Anionen sind beispielsweise Hexafluorophosphat, Hexafluoroantimonat, Tetrachloroaluminat oder Tetrafluoroborat. Diese quaternären Ammonium- und/oder Phosphoniumsalze sind bereits unterhalb von 90°C, vorzugsweise unterhalb von 85°C und besonders bevorzugt unterhalb von 50°C flüssig. In ihnen liegt der Hydroformylierungskatalysator gelöst vor.

Der Hydroformylierungskatalysator enthält als aktives Metall Kobalt, Rhodium, Iridium, Ruthenium, Palladium oder Platin und als Ligand ein tertiäres Phosphin oder tertiäres sulfoniertes Phosphin, ein tertiäres Arsin, tertiäres Stilbin oder ein Phosphit. Nach der Lehre von EP-A-0 776 880 beträgt das Molverhältnis Ligand zu Metall 9,5.

Geeignete, die aktiven Metalle enthaltende Verbindungen, aus denen sich unter den Reaktionsbedingungen der Hydroformylierungskatalysator bildet, sind beispielsweise Rhodiumacetylacetonatdicarbonyl oder das Rhodiumcarbonyl Rh₆(CO)₁₆.

Besonders bevorzugt wird die Hydroformylierungsreaktion zwischen 30 und 90°C durchgeführt.

Auch nach Angew. Chem. 1995, 107 Nr.23/24 Seiten 2941 bis 2943 lassen sich Hydroformylierungsreaktionen unter Verwendung bei Raumtemperatur flüssiger 1,3 Dialkylimidazoliumsalze als katalysatorhaltiges, nicht mit dem organischen Reaktionsgemisch mischbares Lösungsmittel durchführen. Dabei wird Rhodiumdicarbonylacetylacetonat als Katalysatorvorstufe zu einer Lösung von Triphenylphosphin in BMI^{⊕} Hexafluorophosphat gegeben , wobei das Molverhältnis Phosphor (III) zu Rhodium von 3 bis 10 variieren kann. Unter Zugabe von Synthesegas mit einer Zusammensetzung von Wasserstoff zu Kohlenmonoxid im Volumenverhältnis von 1:1, wird der Katalysator präformiert. Nach Zugabe von n-Penten-1 erfolgt die Umsetzung mit Synthesegas gleicher Zusammensetzung bei einer Temperatur von 80 °C. Auch in diesem Falle läßt sich die organische Produktphase in einfacher Weise von der katalysatorhaltigen nichtwäßrigen ionogenen Flüssigkeit durch Abdekantieren abtrennen.

Die bekannten Verfahren zur Hydroformylierung von Olefinen sind durch die Verwendung einer nichtwäßrigen ionogenen Flüssigkeit, die als Lösungsmittel für den katalytisch wirksamen Metallkomplex dient, charakterisiert. Durch die Verwendung der nichtwäßrigen ionogenen Flüssigkeit als Lösungsmittel werden zusätzliche, nicht als Liganden dienende Anionen ,z.B. Hexafluoroantimonat oder Hexafluorophosphat, in den Hydroformylierungsprozeß eingebracht.

Weiterhin lehrt der aus Angew. Chem. 1995, 107 Nr.23/24 Seiten 2941 bis 2943 und EP-A-0 776 880 bekannte Stand der Technik ein Molverhältnis Phosphor zu Rhodium von 3 bis 10. Höhere Molverhältnisse von Phosphor zu Rhodium offenbart der Stand der Technik nicht. Ein höheres Molverhältnis Phosphor zu Rhodium führt vermutlich zu einem Ausfallen oder zu einem verstärkten Austrag des Phosphinliganden aus der offenbarten nichtwäßrigen ionogen Flüssigkeit.

Nachteilig bei den bekannten Verfahren ist neben dem Austrag des Phosphinliganden der Austrag des katalytisch aktiven Metalls aus der nichtwäßrigen ionogenen Flüssigkeit in die organische Phase. Nach dem Stand der Technik läßt sich dieser Nachteil umgehen, wenn an Stelle von neutralen Liganden, z.B. von Triphenylphosphin, geladene Liganden, z.B. mono- oder trisulfoniertes Triphenylphosphin, verwendet werden, da zu erwarten ist, daß geladene Liganden die Löslichkeit der katalytisch aktiven Metallverbindungen in der nichtwäßrigen ionogenen Flüssigkeit erhöhen. Auch wenn hierdurch der Austrag des katalytisch aktiven Metalls durch Verwendung geladener Liganden reduziert werden konnte, verringern sich jedoch die Ausbeuten an Aldehyden auf nur noch 16 bis 33 % (Angew. Chem. 1995, 107 Nr.23/24 Seiten 2941 bis 2943, EP-A-0 776 880).

Es bestand daher die Aufgabe ein Verfahren zu entwickeln, das unter Verwendung einer nichtwäßrigen ionogenen Ligandflüssigkeit nach Zusatz eines katalytisch wirkenden Metalls und/oder dessen Verbindung die gewünschten Aldehyde auf einfachem Wege und in hohen Ausbeuten liefert.

Die Erfindung besteht in einem zweiphasigen Verfahren zur Herstellung von Aldehyden durch Umsetzung von Monoolefinen, nichtkonjugierten Polyolefinen, Cycloolefinen oder Derivaten dieser Verbindungsklasse mit 2 bis 20 Kohlenstoffatomen mit Kohlenmonoxid und Wasserstoff bei Temperaturen von 20 bis 150°C und Drücken von 0,1 bis 20 MPa, charakterisiert durch das Vorliegen einer organischen aldehydhaltigen Phase und einer darin nicht löslichen, nichtwäßrigen ionogenen Ligandflüssigkeit der allgemeinen Formel (Q^{⊕})ₐA^{a-} mit mindestens einer Rhodiumverbindung, dadurch gekennzeichnet, daß Q^{⊕} für quaternäre Ammoniumund/oder Phosphonium-Kationen der allgemeinen Formeln 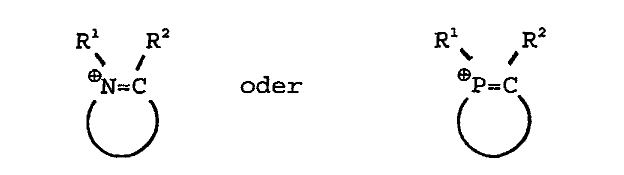 steht, die 1, 2 oder 3 Stickstoffatome und/oder Phosphoratome im Ring tragen, wobei R¹,R² gleich oder verschieden sind und Wasserstoff oder einen geradkettigen oder verzweigten Kohlenwasserstoffrest mit 1 bis 20 Kohlenstoffatomen bedeuten und in denen die Heterocyclen aus 4 bis 10 Ringatomen bestehen; oder dass Q^{⊕} für quaternäre Ammonium- und/oder Phosphonium-Kationen der allgemeinen Formeln R¹R^{2⊕}N=CR³-X-R³C=^{⊕}NR¹R² oder R¹R^{2⊕}P=CR³-X-R³C=^{⊕}PR¹R² steht, wobei R¹,R² und R³ gleich oder verschieden sind und Wasserstoff oder einen geradkettigen oder verzweigten Kohlenwasserstoffrest mit 1 bis 20 Kohlenstoffatomen und X einen Alkylen- oder Phenylenrest bedeutet; oder dass Q^{⊕} für das N-Butylpyridinium-, N-Ethylpyridinium-, 1-n-Butyl-3-methylimidazolium-, Diethylpyrazolium-, 1-Ethyl-3-methylimidazolium-, Pyridinium-Kation, für das Tricyclodecan Diammonium-Kation oder das N,N'-Dimethyl-Tricyclodecan Diammonium-Kation steht; oder dass Q^{⊕} für quaternäre Ammonium- und/oder Phosphonium-Kationen der allgemeinen Formeln R¹R²R³N^{⊕} (̵ X )̵ N^{⊕}R⁴R⁵R⁶ oder R¹R²R³P^{⊕} (̵ X )̵ P^{⊕}R⁴R⁵R⁶ steht, in der R¹, R², R³, R⁴, R⁵ und R⁶ gleich oder verschieden sind und Wasserstoff oder einen geradkettigen oder verzweigten Kohlenwasserstoffrest mit 1 bis 20 Kohlenstoffatomen bedeuten, und X für 1,2-Phenylen, 1,3-Phenylen, 1,4-Phenylen oder für einen Alkylenrest (̵CHR⁷)̵_{b}, wobei R⁷ gleich Wasserstoff oder ein Kohlenwasserstoffrest mit 1 bis 5 Kohlenstoffatomen ist, und b eine ganze Zahl zwischen 1 bis 8 ist, steht und A^{a-} ein Triarylphosphin der allgemeinen Formel 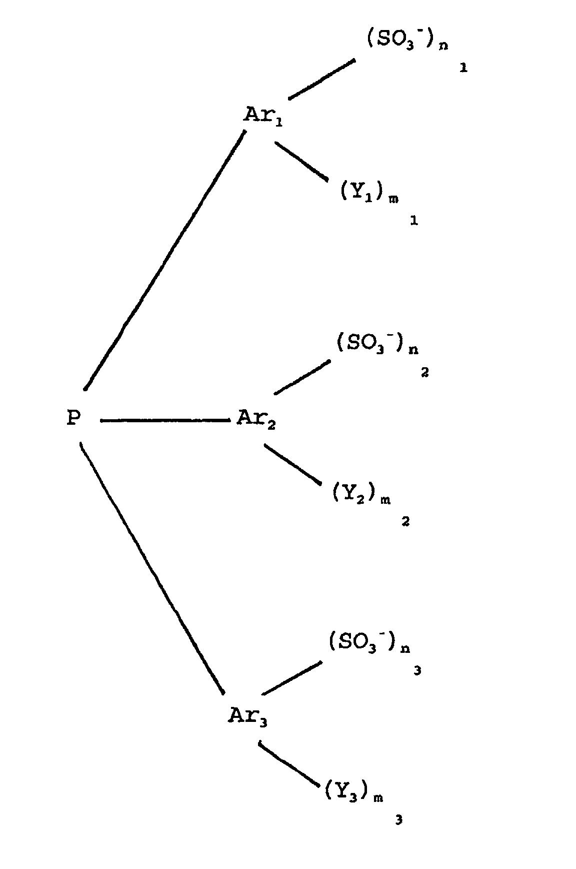 ist, in der Ar¹, Ar² und Ar³ gleiche oder verschiedene Arylgruppen mit 6 bis 14 Kohlenstoffatomen bedeuten, die Substituenten Y₁, Y₂ und Y₃ gleiche oder verschiedene geradkettige oder verzweigte Alkyl- oder Alkoxyreste mit 1 bis 4 Kohlenstoffatomen bedeuten, Chlor, Brom, die Hydroxyl-, Cyanid- oder Nitrogruppe bedeuten, ferner für die Aminogruppe der Formel NR⁸R⁹ stehen, in der die Substituenten R⁸ und R⁹ gleich oder verschieden sind und Wasserstoff, geradkettige oder verzweigte Alkylgruppen mit 1 bis 4 Kohlenstoffatomen bedeuten, in der m₁, m₂ und m₃ gleich oder verschieden sind und ganze Zahlen von 0 bis 5 bedeuten; in der n₁, n₂ und n₃ gleich oder verschieden sind und für ganze Zahlen von 0 bis 3 stehen, wobei mindestens eine der Zahlen n₁, n₂ und n₃ gleich oder größer 1 ist, und in der a gleich n₁+n₂+n₃ ist, und daß über die stöchiometrisch erforderliche Menge zur Bildung von (Q^{⊕})ₐ A^{a-} hinaus ein Überschuß an aus Q^{⊕} abgeleiteten Aminen und/oder Phosphinen von bis zu 5 Äquivalenten oder ein Überschuß an Alkali- und/oder Erdalkalisalzen der Triarylphosphine A^{a-} von bis zu 5 Äquivalenten vorliegt und daß je mol Rhodium 2 bis 1000 mol Phosphor(III) eingesetzt werden, und daß die organische aldehydhaltige Phase und die rhodiumhaltige nichtwäßrige ionogene Ligandflüssigkeit durch Phasentrennung voneinander getrennt werden und die abgetrennte rhodiumhaltige nichtwäßrige ionogene Ligandflüssigkeit vollständig oder teilweise in den Hydroformylierungsreaktor zurückgeführt wird.

Überraschenderweise wurde gefunden, daß sich die erfindungsgemäßen nichtwäßrigen ionogenen Ligandflüssigkeiten nach Zugabe von Rhodium und/oder dessen Verbindungen als Katalysatorsystem für die Hydroformylierung von Olefinen oder olefinisch ungesättigten Verbindungen in hervorrangender Weise eignen.

Dabei wurde gefunden, daß die Verwendung der erfindungsgemäßen nichtwäßrigen ionogenen Ligandflüssigkeiten in Hydroformylierungsprozessen die Verwendung eines hohen Molverhältnisses von Phosphor zu Rhodium von bis zu 1000 zu 1 gestattet.

Ein hoher Überschuß an Liganden, z.B. an sulfoniertem Triphenylphosphin, wirkt stabilisierend auf die katalytisch wirksamen Metallkomplexe während des Katalysecyclus.

Wenn im folgenden von Katalysatorsystem gesprochen wird, so ist darunter die nichtwäßrige ionogene Ligandflüssigkeit zusammen mit den katalytisch aktiven Rhodiumverbindungen gemeint.

Stabilisierte Katalysatorsysteme zeichnen sich durch geringe Austragsraten an Rhodium aus und erlauben ein häufiges Zurückführen des gebrauchten Katalysatorsystems in den Hydroformylierungsprozeß, ohne daß ein Absinken der Katalysatoraktivität und -selektivität beobachtet wird. Stabilisierte Katalysatorsysteme zeigen daher höhere Ausbeuten an Aldehyden und verlängerte Katalysatorstandzeiten als nicht stabilisierte Katalysatorsysteme.

Bei der Verwendung nichtwäßriger ionogener Flüssigkeiten und nichtwäßriger ionogener Ligandflüssigkeiten ist die Verlängerung der Katalysatorstandzeiten, wie sie bekanntermaßen durch stabilisierte Katalysatorsysteme erzielt werden kann, von besonderer Bedeutung, da die erschöpfte Katalysatorphase nach dem Ausschleusen aus dem Hydroformylierungsprozeß eine hohe Salzfracht darstellt, die aufwendig aufgearbeitet und/oder entsorgt werden muß. Ein Erschöpfen des Katalysatorsystems zeigt sich in einem Absinken der Katalysatoraktivität und -selektivität unter ein wirtschaftlich vertretbares Maß an. Aktivitäts- und Selektivitätsabnahme werden z.B. durch das Anreichern von Katalysatorabbauprodukten verursacht. Führt man daher übergangsmetallkatalysierte Zweiphasen-Prozesse in nichtwäßrigen ionogenen Flüssigkeiten durch, ist eine zu rasche Erschöpfung des Katalysatorsystems, die ein nachfolgendes Ausschleusen aus dem Hydroformylierungsprozeß erfordert, besonders nachteilig. Mit den erfindungsgemäßen nichtwäßrigen ionogenen Ligandenflüssigkeiten gelingt es, ein Molverhältnis von Phosphor zu Rhodium von bis zu 1000 zu 1 einzustellen, was bekanntlich die Stabilität und damit die Lebensdauer von phosphansubstituierten Katalysatoren erhöht.

Es ist anzunehmen, daß sich in Gegenwart von Kohlenmonoxid und Wasserstoff die katalytisch aktiven Rhodiumverbindungen aus dem Rhodium, das entweder in metallischer Form oder als gängige Rhodiumverbindung zugesetzt wird, und der nichtwäßrigen ionogenen Ligandflüssigkeit bilden. Die nichtwäßrige ionogene Ligandflüssigkeit und die katalytisch aktive Rhodiumverbindung bilden das Katalysatorsystem.

Durch die Verwendung der erfindungsgemäßen nichtwäßrigen ionogenen Ligandflüssigkeiten in Hydroformylierungsreaktionen kann auf die Zugabe zusätzlicher, nicht als Liganden dienender Anionen in solchen Prozessen verzichtet werden.
Die erfindungsgemäßen, nicht wäßrigen ionogenen Ligandflüssigkeiten können über die stöchiometrisch erforderliche Menge zur Bildung von (Q^{⊕})ₐ A^{a-} hinaus einen Überschuß an aus Q^{⊕} abgeleiteten Aminen und/oder Phosphinen oder einen Überschuß an Alkali- und/oder Erdalkalisalzen der Triarylphosphine A^{a-} enthalten. Im allgemeinen liegt der Überschuß über die stöchiometrisch erforderliche Menge zur Bildung von (Q^{⊕})ₐ A^{a-} hinaus bei bis zu 5 Äquivalenten an aus Q^{⊕} abgeleiteten Aminen und/oder Phosphinen oder an Alkali- und/oder Erdalkalisalzen der Triarylphosphine A^{a-}, vorzugsweise liegt dieser Überschuß bei bis zu 1 Äquivalent.

Als Kationen Q^{⊕} für die Herstellung der erfindungsgemäßen nichtwäßrigen ionogenen Ligandflüssigkeiten lassen sich quaternäre Ammonium- und/oder Phosphonium-Kationen der allgemeinen Formeln 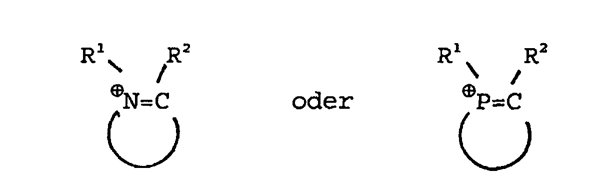 verwenden, die 1, 2 oder 3 Stickstoff- und/oder Phosphoratome im Ring tragen. Die Heterocyclen bestehen aus 4 bis 10, vorzugsweise 5 bis 6 Ringatomen. R¹ und R² sind gleich oder verschieden und bedeuten Wasserstoff oder einen geradkettigen oder verzweigten Kohlenwasserstoffrest mit 1 bis 20 Kohlenstoffatomen, beispielsweise einen Alkyl-, Alkenyl-, Cycloalkyl-, Alkylaryl-, Aryloder Aralkylrest.

Weiterhin sind quaternäre Ammonium- und Phosphonium Kationen der allgemeinen Formel

R¹R^{2⊕}N=CR³-X-R³C=^{⊕}NR¹R²

R¹R^{2⊕}P=CR³-X-R³C=^{⊕}PR¹R²

geeignet, in denen R¹,R²,R³ gleich oder verschieden sind und Wasserstoff oder einen geradkettigen oder verzweigten Kohlenwasserstoffrest mit 1 bis 20 Kohlenstoffatomen, beispielsweise einen Alkyl-, Alkenyl-, Cycloalkyl-, Alkylaryl-, Aryl- oder Aralkylrest bedeuteten und X einen Alkylen- oder Phenylenrest bedeutet. R¹,R²,R³ sind beispielsweise die Methyl, Ethyl-, Propyl-, Isopropyl-, Butyl-, sekundär-Butyl-, tertiär-Butyl-, Amyl-, Methylen-, Ethyliden-, Phenyl-, oder Benzylgruppe. X steht für 1,2-Phenylen, 1,3-Phenylen, 1,4-Phenylen oder für einen Alkylenrest, beispielsweise für den Methylen-, Ethylen-, Propylen- oder 1,4-Butylenrest.

Als Kation Q^{⊕} für die Herstellung der erfindungsgemäßen nichtwäßrigen ionogen Ligandflüssigkeit eignet sich auch das N-Butylpyridinium-, N-Ethylpyridinium-, 1-n-Butyl-3-methylimidazolium-, Diethylpyrazolium-, 1-Ethyl-3-methylimidazolium- oder das Pyridinium-Kation.

Als weitere Kationen Q^{⊕} eignen sich für die Herstellung der erfindungsgemäßen nichtwäßrigen ionogenen Ligandflüssigkeiten quaternäre Ammonium- und/oder Phosphonium-Kationen der allgemeinen Formel

R¹R²R³N^{⊕} (̵ X )̵ N^{⊕}R⁴R⁵R⁶ (quaternäre Diamine)

R¹R²R³P^{⊕} (̵ X )̵ P^{⊕}R⁴R⁵R⁶ (quaternäre Diphosphine)

in der R¹, R², R³, R⁴, R⁵ und R⁶ gleich oder verschieden sind und Wasserstoff, geradkettige oder verzweigte Kohlenwasserstoffreste mit 1 bis 20 Kohlenstoffatomen bespielsweise einen Alkyl-, Alkenyl-, Cycloalkyl-, Alkylaryl-, Aryl- oder Aralkylrest bedeuten, und X für 1,2-Phenylen, 1,3-Phenylen, 1,4-Phenylen oder für einen Alkylenrest (̵CHR⁷)̵_{b}, wobei R⁷ gleich Wasserstoff oder ein Kohlenwasserstoffrest mit 1 bis 5 Kohlenstoffatomen, beispielsweise Methyl, Ethyl, n-Propyl, i-Propyl, n-Butyl, i-Butyl und t-Butyl ist, und b eine ganze Zahl zwischen 1 bis 8 ist, steht. Beispiele für X sind der Methylen, Ethylen, Propylen, Butylen oder 1,4-Phenylenrest.

Die quaternären Ammonium-Kationen der allgemeinen Formel R¹R²R³N^{⊕} (̵ X )̵ N^{⊕}R⁴R⁵R⁶ werden im folgenden quaternären Diamine genannt.

Zu den quaternären Diaminen, die sich zur Herstellung der erfindungsgemäßen nichtwäßrigen ionogenen Ligandflüssigkeiten eignen, zählen solche quaternäre Diamine der allgemeinen Formel R¹R²R³N^{⊕} (̵CHR⁷)̵_{b} N^{⊕}R⁴R⁵R⁶, in der R¹, R², R³, R⁴, R⁵ und R⁶ gleich oder verschieden sind und für Wasserstoff, n-Butyl, n-Pentyl, n-Hexyl, n-Heptyl, i-Heptyl, n-Octyl, i-Octyl, n-Nonyl, i-Nonyl, n-Decyl, i-Decyl, n-Undecyl, i-Undecyl, n-Dodecyl oder i-Dodecyl stehen, R⁷ Wasserstoff, Methyl oder Ethyl bedeutet, und in denen b gleich 2, 3, 4, 5 oder 6 ist.

Besonders geeignet für die Herstellung der erfindungsgemäßen nichtwäßigen ionogenen Ligandflüssigkeiten sind solche quaternären Diamine, die sich von 1-Amino-3-dialkylaminopropanen der allgemeinen Formel

R¹R²N-CH₂-CH₂-CH₂-NH₂

in der R¹ und R² gleiche oder verschiedene geradkettige oder verzweigte Alkylreste mit 4 bis 20 Kohlenstoffatomen, beispielsweise die n-Butyl, n-Pentyl-, n-Hexyl-, n-Heptyl-, i-Heptyl-, n-Octyl-, i-Octyl-, i-Nonyl-, n-Nonyl-, n-Decyl-, i-Decyl-, n-Undecyl-, i-Undecyl-, n-Dodecyl- oder i-Dodecyl- bedeuten, ableiten lassen.

Insbesondere vorteilhaft lassen sich die erfindungsgemäßen nichtwäßrigen ionogenen Ligandflüssigkeiten herstellen, wenn man 1-Amino-3-(di-n-heptyl)-aminopropan, 1-Amino-3-(di-i-heptyl)-aminopropan, 1-Amino-3-(di-noctyl)-aminopropan, 1-Amino-3-(di-i-octyl)-aminopropan, 1-Amino-3-(di-n-nonyl)-aminopropan ,1-Amino-3-(di-inonyl)-aminopropan, 1-Amino-3-(di-n-undecyl)-aminopropan, 1-Amino-3-(di-i-undecyl)aminopropan, 1-Amino-3-(di-ndodecyl)aminopropan oder 1-Amino-3-(di-i-dodecyl)aminopropan zur Herstellung der quaternären Diamine verwendet.

Die Herstellung der 1-Amino-3-dialkylaminopropane erfolgt durch Umsetzung der N,N-(dialkyl)amine der allgemeinen Formel,

R¹R²NH

in der R₁ und R₂ gleiche oder verschiedene geradkettige oder verzweigte Alkylreste mit 4 bis 20 Kohlenstoffatomen, insbesondere die n-Butyl, n-Pentyl-, n-Hexyl-, n-Heptyl-, i-Heptyl-, n-Octyl-, i-Octyl-, i-Nonyl-, n-Nonyl-, n-Decyl-, i-Decyl- n-Undecyl-, i-Undecyl, n-Dodecyl- oder i-Dodecylgruppe bedeuten, mit Acrylnitril nach bekannten Verfahren (vgl. Ullmann's Encyclopedia of Industrial Chemistry Vol. A2, 1985).

Als weitere von Q^{⊕} abgeleitete Diamine lassen sich Tricyclodecan Diamin und N,N'-Dimethyl-Tricyclodecan Diamin verwenden.

Die zur Durchführung des erfindungsgemäßen Verfahrens verwendeten Triarylphosphine A^{a-} lassen sich aus den nach DE-OS-26 27 354 bekannten Alkali- und/oder Erdalkalisalzen der Triarylphosphine der allgemeinen Formel 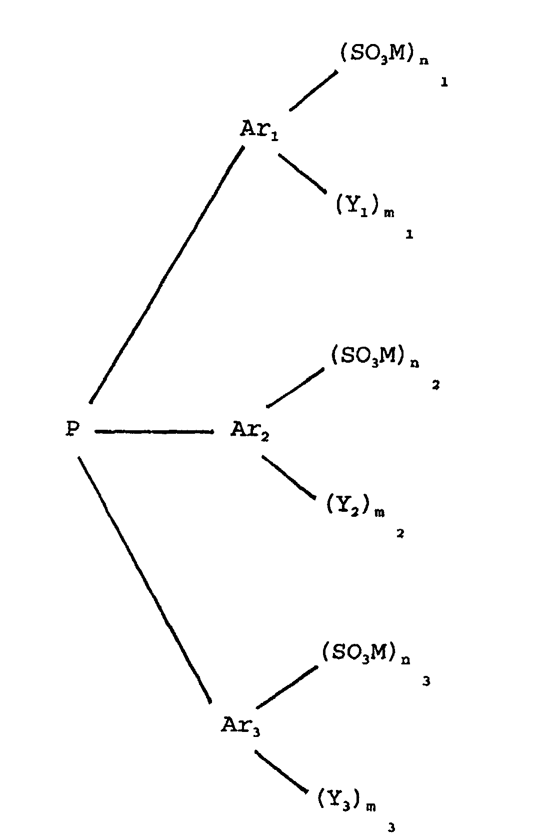 erhalten, in der Ar¹, Ar² und Ar³ gleiche oder verschiedene Arylgruppen mit 6 bis 14 Kohlenstoffatomen bedeuten, die Substituenten Y₁, Y₂ und Y₃ gleiche oder verschiedene geradkettige oder verzweigte Alkyl- oder Alkoxyreste mit 1 bis 4 Kohlenstoffatomen, Chlor, Brom, die Hydroxyl-, Cyanid- oder Nitrogruppe bedeuten, ferner für die Aminogruppe der Formel NR⁸R⁹ stehen, in der die Substituenten R⁸ und R⁹ gleich oder verschieden sind und Wasserstoff, geradkettige oder verzweigte Alkylgruppen mit 1 bis 4 Kohlenstoffatomen bedeuten, in der M für Lithium, Natrium, Kalium, Magnesium, Calcium oder Barium steht, in der m₁, m₂ und m₃ gleich oder verschieden sind und ganze Zahlen von 0 bis 5 bedeuten, in der n₁, n₂ und n₃ gleich oder verschieden sind und für ganze Zahlen von 0 bis 3 stehen, wobei mindestens eine der Zahlen n₁, n₂ und n₃ gleich oder größer 1 ist.

Zu den Triarylphosphinen zählen bevorzugt solche Triarylphosphine, bei denen die Gruppen Ar₁, Ar₂, Ar₃ Phenylgruppen sind, Y₁, Y₂ und Y₃ für die Methyl-, die Ethylgruppe, für die Methoxy-, Ethoxygruppe und/oder für ein Chloratom stehen; und die kationischen Reste M anorganische Kationen von Natrium, Kalium, Calcium und Barium sind. Insbesonders geeignet sind solche Triarylphosphine , bei denen Ar₁, Ar₂ , Ar₃ jeweils eine Phenylgruppe bedeuten, m₁, m₂, m₃ gleich 0 sind, n₁, n₂, und n₃ gleich 0 oder 1 sind und n₁+n₂+n₃ zusammen 1 bis 3 ausmachen, und bei denen die Sulfonat-Gruppen in meta-Stellung stehen.

Besonders eignen sich wäßrige Lösungen von Natrium-, Kalium-, Calcium- oder Bariumsalzen von (Sulfophenyl)-diphenylphosphin, Di-(sulfophenyl)phenylphosphin oder Tri(sulfophenyl)phosphin. Es lassen sich auch Gemische dieser wäßrigen Lösungen verwenden. Es ist jedoch vorteilhaft, eine einzige wäßrige Salzlösung von einem der genannten Alkali- und/oder Erdalkalielemente, insbesondere eine wäßrige Natrium- oder Kaliumsalzlösung, zu verwenden, wobei diese Lösung auch ein Gemisch aus (Sulfophenyl)-diphenylphosphin, Di-(Sulfophenyl)diphenylphosphin, und Tri(sulfophenyl)phosphin enthalten kann.
Ein für die Durchführung des erfindungsgemäßen Hydroformylierungsverfahrens geeignetes Gemisch fällt bei der Sulfonierung von Triphenylphosphin an, wie z. B. aus DE OS 26 27 354 bekannt.

Verwendet man Tricyclodecan Diamin oder N,N-Dimethyl-Tricyclodecan Diamin als Amin zur Herstellung der nichtwäßrigen ionogenen Ligandflüssigkeit, ist es zweckmäßig, ein Gemisch mit einem möglichst hohen Gehalt an Di-(sulfophenyl)phenylphosphin zu verwenden.

Das Verfahren zur Herstellung der nichtwäßrigen ionogenen Ligandflüssigkeit ist Gegenstand einer am gleichen Tag eingereichten Patentanmeldung.

Nach dem erfindungsgemäßen Verfahren lassen sich Monoolefine , nicht konjugierte Polyolefine, cyclische Olefine und Derivate dieser ungesättigten Verbindungen mit 2 bis 20 Kohlenstoffatomen umsetzen. Die Olefine können geradkettig oder verzweigt, die Doppelbindungen end- oder innenständig sein. Beispiele für Olefine, die in dem neuen Verfahren verwendet werden können, sind Ethylen, Propylen, Buten-1, Buten-2, Penten-1, 2-Methylbuten-1, Hexen-1, Hexen-2, Hepten-1, Octen-1, Octen-3, 3-Ethylhexen-1, Decen-1, Undecen-3, 4,4-Dimethylnonen-1, Dicyclopentadien, Vinylcyclohexen, Cyclooctadien, Styrol. Derivate der genannten Olefinarten, die nach der beanspruchten Arbeitsweise hydroformyliert werden können, sind z.B. Alkohole, Aldehyde, Carbonsäuren, Ester, Nitrile und Halogenverbindungen, Allylalkohol, Acrolein, Methacrolein, Crotonaldehyd, Methylacrylat, Ethylcrotonat, Diethylfumarat und Diethylmaleinat, Acrylnitril. Nach dem erfindungsgemäßen Verfahren lassen sich insbesondere wasserempfindliche Olefine und Olefinderivate, wie z.B. Vinylacetat, Vinylpropionat, Vinylbutyrat, Allylacetat, Allylpropionat, Allylbutyrat, Acrylate mit 4 bis 20 Kohlenstoffatomen, Acroleindialkylacetale mit Alkylgruppen von 1 bis 17 Kohlenstoffatomen und andere Olefinderivate, die im Molekül eine hydrolyseempfindliche Gruppe, z.B. die Ester- oder Amidgruppe, enthalten, in guten Ausbeuten hydroformylieren.

Als organische Phase läßt sich das Reaktionsgemisch, bestehend aus dem eingesetzten Olefin und dem bereits gebildeten Aldehyd ohne und mit Lösungsmittelzusatz verwenden. Arbeitet man mit Lösungsmittelzusatz, wird bevorzugt Toluol, o-Xylol, m-Xylol, p-Xylol, Mesitylen, Benzol, Cyclohexan, Tetrahydrofuran, n-Hexan, n-Heptan oder n-Octan verwendet.

Rhodium gelangt entweder als Metall oder als Verbindung zum Einsatz. In metallischer Form verwendet man es entweder als feinverteilte Partikel oder in dünner Schicht auf einem Träger, wie Aktivkohle, Calciumcarbonat, Aluminiumsilikat, Tonerde niedergeschlagen. Als Rhodiumverbindungen eignen sich Salze aliphatischer Mono- und Polycarbonsäuren, wie Rhodium-2-ethylhexanoat, Rhodiumacetat, Rhodiumoxalat, Rhodiumpropionat oder Rhodiummalonat. Weiterhin können Rhodiumsalze anorganischer Wasserstoff- und Sauerstoffsäuren, wie Rhodiumnitrat oder Rhodiumsulfat, die verschiedenen Rhodiumoxide oder auch Rhodiumcarbonylverbindungen wie Rh₃(CO)₁₂ oder Rh₆(CO)₁₆ oder Komplexverbindungen des Rhodiums, z.B. Cyclooctadienylrhodiumverbindungen oder Rhodiumacetylacetonat, eingesetzt werden. Rhodiumhalogenverbindungen kommen wegen des korrosiven Verhaltens der Halogenidionen weniger in Betracht.

Bevorzugt werden Rhodiumoxid und insbesondere Rhodiumacetat und Rhodium-2-ethylhexanoat.
Das Katalysatorsystem kann zunächst in einem Präformierungsschritt gebildet und dann dem Reaktionsgemisch präformiert zugesetzt werden. Dabei wird zu der nichtwäßrigen ionogenen Ligandflüssigkeit die gewünschte Menge Rhodium, vorzugsweise als Rhodium-2-ethylhexanoat-Lösung hinzugegeben und das Reaktionsgemisch bei einer Temperatur von 100 bis 120°C mit Synthesegas bei einem Druck von 0,5 bis 5 MPa über einen Zeitraum bis zu 5 Stunden behandelt. Nach der Präformierung des Katalysators erfolgt die Umsetzung der Olefine mit Wasserstoff und Kohlenmonoxid bei Temperaturen von 20 bis 150°C, bevorzugt 80 bis 140°C und insbesondere 100 bis 125°C und Drücken von 0,1 bis 20 MPa, bevorzugt 1 bis 12 MPa und insbesondere 3 bis 7 MPa.

Der Präformierungsschritt kann auch in Gegenwart eines Lösungsmittels durchgeführt werden, z.B. in Gegenwart von Toluol, o-Xylol, m-Xylol, p-Xylol, Cyclohexan oder Heptan. Bevorzugt wird Toluol oder Cyclohexan als Lösungsmittel.

Die Zusammensetzung des Synthesegases, d.h. das Verhältnis von Kohlenmonoxid zu Wasserstoff, kann in weiten Grenzen variiert werden. Im allgemeinen setzt man Synthesegas ein, in dem das Volumenverhältnis von Kohlenmonoxid zu Wasserstoff 1:1 beträgt oder von diesem Wert nur wenig abweicht.

Mit gleich gutem Erfolg läßt sich das Katalysatorsystem unter Reaktionsbedingungen, also in Gegenwart des Olefins herstellen. Dabei kann das Olefin und Aldehyd als Lösungsmittel für den Katalysator dienen. Arbeitet man mit einem Lösungsmittel, so wird bevorzugt Toluol, o-Xylol, m-Xylol, p-Xylol, Cyclohexan, Mesitylen oder n-Heptan und insbesondere Toluol oder Cyclohexan verwendet.

Die Rhodiumkonzentration beträgt vorzugsweise 3 bis 400 Gew.-ppm und besonders bevorzugt 5 bis 100 Gew.-ppm, bezogen auf die eingesetzte Olefinmenge.

Die sulfonierten Triarylphosphine A^{a-}, die Bestandteil der erfindungsgemäßen nichtwäßrigen ionogenen Ligandflüssigkeit sind, liegen in Bezug auf die eingesetzte Rhodiummenge im Überschuß vor.

Dabei kann man das Verhältnis von Rhodium zu dem sulfonierten Triarylphosphin A^{a-}, auch ausgedrückt als das Verhältnis von Rhodium zu Phosphor(III), in weiten Grenzen variieren und je mol Rhodium im allgemeinen 2 bis 1000 mol Phosphor(III) anwenden. Bevorzugt wird ein Molverhältnis Phosphor(III) zu Rhodium von 3 bis 300 und insbesondere von 20 bis 100.

Die Umsetzung kann sowohl absatzweise als auch kontinuierlich durchgeführt werden. Nach erfolgter Umsetzung liegt eine organische aldehydhaltige obere Phase und das Katalysatorsystem als untere Phase vor, die durch einfache Phasentrennnung voneinander getrennt werden können. Nach erfolgter Phasentrennung läßt sich das Katalysatorsystem wieder in den Hydroformylierungsprozeß zurückführen.

Die folgenden Beispiele erläutern das erfindungsgemäße Verfahren, beschränken es jedoch nicht.

### Beispiel 1:

### Hydroformylierung von n-Hexen in Gegenwart von 1-Amino-3-(di-i-nonyl)-aminopropan/Trisulfophenylphosphin

Gemäß der am gleichen Tag eingereichten Patentanmeldung, auf die hier besonders Bezug genommen wird wurde eine nichtwäßrige ionogene Ligandflüssigkeit aus 1-Amino-3-(di-i-nonyl)aminopropan und Tri-(sulfophenyl)phosphin verwendet. In der eingesetzten nichtwäßrigen ionogenen Ligandflüssigkeit lag ein Überschuß von 0,45 Äquivalenten an den Natriumsalzen der Triarylphosphine vor.

In einem 1 Liter Autoklaven wurden 420 g der nichtwäßrigen ionischen Ligandflüssigkeit gegeben und entsprechend einem Molverhältnis von Phosphor(III) zu Rhodium von 136 zu 1 mit einer Rhodium-2-ethylhexanoatlösung in 2-Ethylhexanol versetzt. Anschließend wurde n-Hexen in einer solchen Menge zugegeben, daß die Rhodiumkonzentration, bezogen auf die eingesetzte Olefinmenge, 400 Gew.-ppm betrug. Die Umsetzung mit Synthesegas erfolgte bei einer Temperatur von 125°C und einem Druck von 4 MPa über einen Zeitraum von 1,5 Stunden. Danach wurde die Reaktion abgebrochen und der Autoklav entspannt und die organische Phase (das Reaktionsprodukt) über einen Tauchstutzen entnommen und analysiert. Der Olefinumsatz beträgt 71 %, das Verhältnis von n-Heptanal zu 2-Methylhexanal beträgt 69:31. Das Katalysatorsystem wurde zweimal rezirkuliert, ohne daß sich Umsatz und Selektivität verschlechterten.

### Beispiel 2:

### Hydroformylierung von n-Hexen in Gegenwart von 1-Amino-3-(di-n-octyl)-aminopropan/Trisulfophenylphosphin;

Gemäß der am gleichen Tag eingereichten Patentanmeldung, auf die hier besonders Bezug genommen wird wurde eine nichtwäßrige ionogene Ligandflüssigkeit aus 1-Amino-3-(di-n-octyl)aminopropan und Tri-(sulfophenyl)phosphin verwendet. In der eingesetzten nichtwäßrigen ionogenen Ligandflüssigkeit lag ein Überschuß von 1,1 Äquivalenten an 1-Amino-3-(di-noctyl)aminopropan vor.

485 g der nichtwäßrigen ionogenen Ligandflüssigkeit wurden in einem 1 Liter Autoklaven mit 306 g Cyclohexan versetzt und mit einer Lösung von Rhodium-2-ethylhexanoat in 2-Ethylhexanol entsprechend einem Molverhältnis von Phosphor(III) zu Rhodium von 64 zu 1 versetzt. Anschließend wurde n-Hexen in einer solchen Menge zugegeben, daß die Rhodiumkonzentration, bezogen auf die eingesetzte Olefinmenge, 400 Gew.-ppm betrug. Die Umsetzung mit Synthesegas erfolgte bei einer Temperatur von 125°C und einem Druck von 2,5 MPa über einen Zeitraum von 45 Minuten. Danach wurde die Reaktion abgebrochen und der Autoklav entspannt und das Reaktionsprodukt über einen Tauchstutzen entnommen und analysiert. Der Olefinumsatz beträgt 97 %, das Verhältnis von n-Heptanal zu 2-Methylhexanal beträgt 66:34.

### Beispiel 3:

### Hydroformylierung von n-Hexen in Gegenwart von 1-Amino-3-(di-n-octyl) aminopropan/Trisulfophenylphosphin; Rezirkulierungsversuche

Das gemäß Beispiel 2 verwendete Katalysatorsystem wurde für die Hydrofomylierung von n-Hexen unter den gleichen Hydroformylierungsbedingungen wie in Beispiel 2 wiederholt eingesetzt. Wie aus Tabelle 1 sichtlich, wird selbst nach mehrfacher Rezirkulierung des Katalysatorsystems eine hohe Ausbeute an den Aldehyden beobachtet. Überraschenderweise zeigt sich, daß bei der Verwendung der erfindingsgemäßen nichtwäßrigen ionogenen Ligandflüssigkeiten die Austragsraten an Rhodium (Menge an Rhodium, die in 1 kg organischem Reaktionsprodukt gefunden werden) bei mehrfacher Rezirkulierung stetig abnehmen.

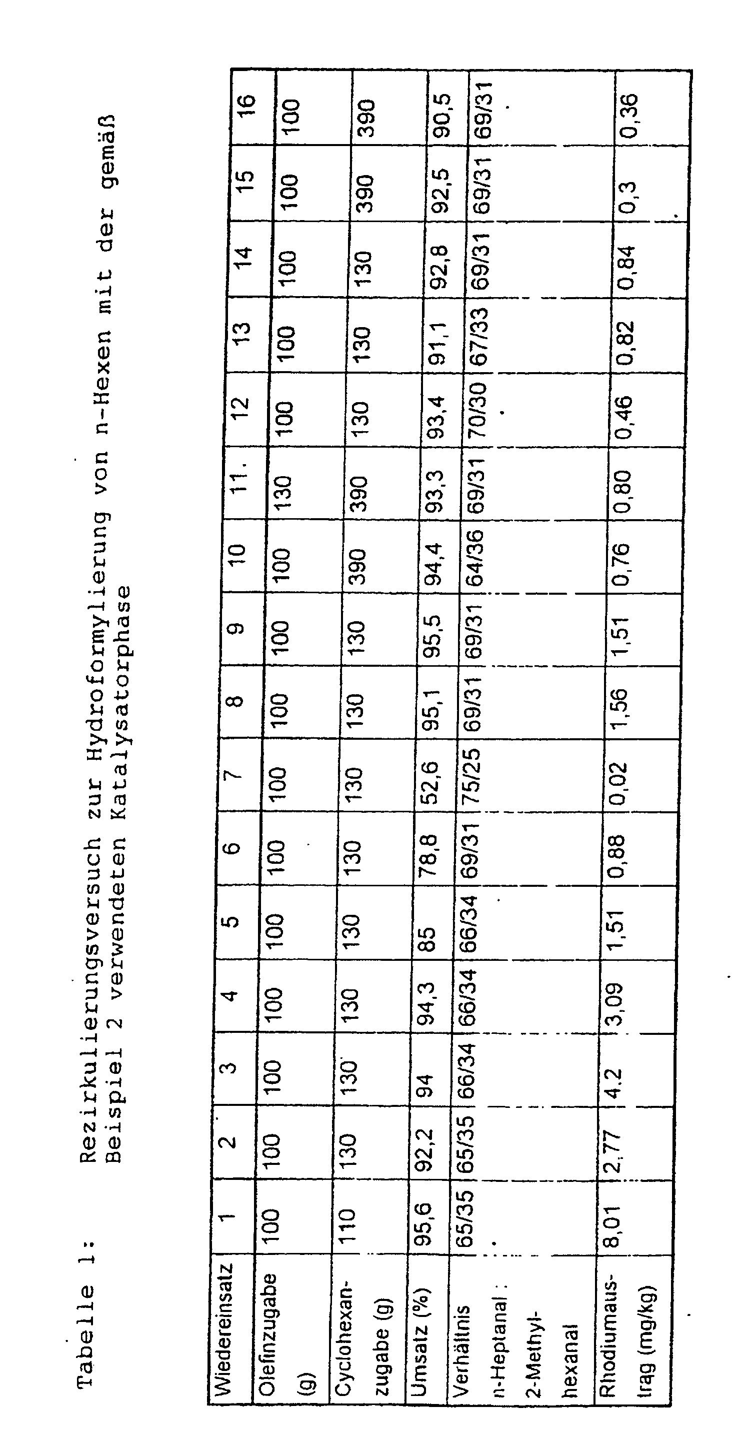

## Patentansprüche

1. Zweiphasiges Verfahren zur Herstellung von Aldehyden durch Umsetzung von Monoolefinen, nichtkonjugierten Polyolefinen, Cycloolefinen oder Derivaten dieser Verbindungsklasse mit 2 bis 20 Kohlenstoffatomen mit Kohlenmonoxid und Wasserstoff bei Temperaturen von 20 bis 150°C und Drücken von 0,1 bis 20 MPa, charakterisiert durch das Vorliegen einer organischen aldehydhaltigen Phase und einer darin nicht löslichen, nichtwäßrigen ionogenen Ligandflüssigkeit der allgemeinen Formel (Q^{⊕})ₐA^{a-} mit mindestens einer Rhodiumverbindung, **dadurch gekennzeichnet, daß** Q^{⊕} für quaternäre Ammonium- und/oder Phosphonium-Kationen der allgemeinen Formeln 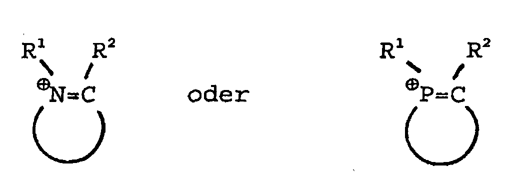 steht, die 1, 2 oder 3 Stickstoffatome und/oder Phosphoratome im Ring tragen, wobei R¹,R² gleich oder verschieden sind und Wasserstoff oder einen geradkettigen oder verzweigten Kohlenwasserstoffrest mit 1 bis 20 Kohlenstoffatomen bedeuten und in denen die Heterocyclen aus 4 bis 10 Ringatomen bestehen; oder dass Q^{⊕} für quaternäre Ammonium- und/oder Phosphonium-Kationen der allgemeinen Formeln R¹R^{2⊕}N-CR³-X-R³C=^{⊕}NR¹R² oder R¹R^{2⊕}P=CR³-X-R³C=^{⊕}PR¹R² steht, wobei R¹, R² und R³ gleich oder verschieden sind und Wasserstoff oder einen geradkettigen oder verzweigten Kohlenwasserstoffrest mit 1 bis 20 Kohlenstoffatomen und X einen Alkylen- oder Phenylenrest bedeutet; oder dass Q^{⊕} für das N-Butylpyridinium-, N-Ethylpyridinium-, 1-n-Butyl-3-methylimidazolium-, Diethylpyrazolium-, 1-Ethyl-3-methylimidazolium-, Pyridinium-Kation, für das Tricyclodecan Diammonium-Kation oder das N,N'-Dimethyl-Tricyclodecan Diammonium-Kation steht; oder dass Q^{⊕} für quaternäre Ammonium- und/oder Phosphonium-Kationen der allgemeinen Formeln R¹R²R³N^{⊕} (̵ X )̵ N^{⊕}R⁴R⁵R⁶ oder R¹R²R³P^{⊕} (̵ X )̵ P^{⊕}R⁴R⁵R⁶ steht, in der R¹, R², R³, R⁴, R⁵ und R⁶ gleich oder verschieden sind und Wasserstoff oder einen geradkettigen oder verzweigten Kohlenwasserstoffrest mit 1 bis 20 Kohlenstoffatomen bedeuten, und X für 1,2-Phenylen, 1,3-Phenylen, 1,4-Phenylen oder für einen Alkylenrest (̵CHR⁷)̵_{b}, wobei R⁷ gleich Wasserstoff oder ein Kohlenwasserstoffrest mit 1 bis 5 Kohlenstoffatomen ist, und b eine ganze Zahl zwischen 1 bis 8 ist, steht und A^{a-} ein Triarylphosphin der allgemeinen Formel 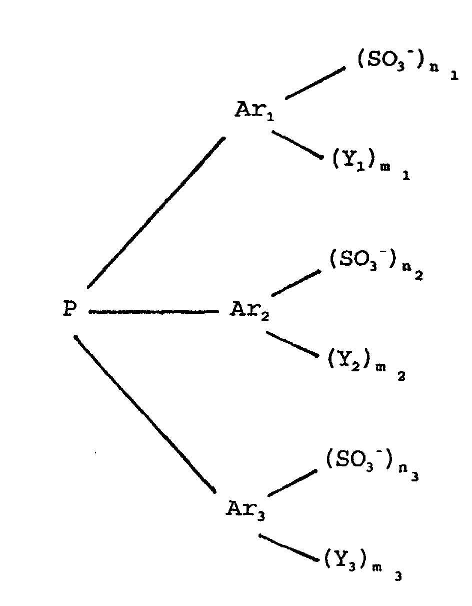 ist, in der Ar¹, Ar² und Ar³ gleiche oder verschiedene Arylgruppen mit 6 bis 14 Kohlenstoffatomen bedeuten, die Substituenten Y₁, Y₂ und Y₃ gleiche oder verschiedene geradkettige oder verzweigte Alkyloder Alkoxyreste mit 1 bis 4 Kohlenstoffatomen bedeuten, Chlor, Brom, die Hydroxyl-, Cyanid- oder Nitrogruppe bedeuten, ferner für die Aminogruppe der Formel NR⁸R⁹ stehen, in der die Substituenten R⁸ und R⁹ gleich oder verschieden sind und Wasserstoff, geradkettige oder verzweigte Alkylgruppen mit 1 bis 4 Kohlenstoffatomen bedeuten, in der m₁, m₂ und m₃ gleich oder verschieden sind und ganze Zahlen von 0 bis 5 bedeuten; in der n₁, n₂ und n₃ gleich oder verschieden sind und für ganze Zahlen von 0 bis 3 stehen, wobei mindestens eine der Zahlen n₁, n₂ und n₃ gleich oder größer 1 ist, und in der a gleich n₁+n₂+n₃ ist, und daß über die stöchiometrisch erforderliche Menge zur Bildung von (Q^{⊕})ₐ A^{a-} hinaus ein Überschuß an aus Q^{⊕} abgeleiteten Aminen und/oder Phosphinen von bis zu 5 Äquivalenten oder ein Überschuß an Alkali- und/oder Erdalkalisalzen der Triarylphosphine A^{a-} von bis zu 5 Äquivalenten vorliegt, und daß je mol Rhodium 2 bis 1000 mol Phosphor(III) eingesetzt werden, und daß die organische aldehydhaltige Phase und die rhodiumhaltige nichtwäßrige ionogene Ligandflüssigkeit durch Phasentrennung voneinander getrennt werden und die abgetrennte rhodiumhaltige nichtwäßrige ionogene Ligandflüssigkeit vollständig oder teilweise in den Hydroformylierungsreaktor zurückgeführt wird.

2. Verfahren nach Anspruch 1, **dadurch gekennzeichnet, daß** über die stöchiometrisch erforderliche Menge zur Bildung von (Q^{⊕})ₐ A^{a-} hinaus ein Überschuß an aus Q^{⊕} abgeleiteten Aminen und/oder Phosphinen von bis zu 1 Äquivalent oder ein Überschuß an Alkali- oder Erdalkalisalzen der Triarylphosphine A^{a-} von bis zu 1 Äquivalent vorliegt.

3. Verfahren nach Anspruch 1 oder 2, **dadurch gekennzeichnet, daß** R¹, R², R³, R⁴, R⁵ und R⁶ gleich oder verschieden sind und für Wasserstoff, n-Butyl, n-Pentyl, n-Hexyl, n-Heptyl, i-Heptyl, n-Octyl, i-Octyl, n-Nonyl, i-Nonyl, n-Decyl, i-Decyl, n-Undecyl, i-Undecyl, n-Dodecyl oder i-Dodecyl stehen, R⁷ Wasserstoff, Methyl oder Ethyl bedeutet und b gleich 2, 3, 4, 5 oder 6 ist.

4. Verfahren nach Anspruch 3, **dadurch gekennzeichnet, daß** R¹ und R² gleich oder verschieden sind und für n-Butyl, n-Pentyl, n-Hexyl, n-Heptyl, i-Heptyl, n-Octyl, i-Octyl, n-Nonyl, i-Nonyl, n-Decyl, i-Decyl, n-Undecyl, i-Undecyl, n-Dodecyl oder i-Dodecyl stehen, R³, R⁴, R⁵ und R⁶ Wasserstoff bedeuten, R⁷ gleich Wasserstoff ist und b gleich 3 ist.

5. Verfahren nach einem oder mehreren der Ansprüche 1 bis 4, **dadurch gekennzeichnet, daß** je mol Rhodium 3 bis 300, insbesondere 20 bis 100 mol Phosphor (III) eingesetzt werden.

6. Verfahren nach einem der Ansprüche 1 bis 5, **dadurch gekennzeichnet, daß** die Rhodiumkonzentration 2 bis 1000 Gew.-ppm, vorzugsweise 3 bis 400 Gew.-ppm und insbesondere 5 bis 100 Gew.-ppm, bezogen auf die eingesetzte Olefinmenge, beträgt.

7. Verfahren nach einem der Ansprüche 1 bis 6, **dadurch gekennzeichnet, daß** die Umsetzung bei 20 bis 150°C, vorzugsweise bei 80 bis 140°C und insbesondere 100 bis 125°C erfolgt.

8. Verfahren nach einem der Ansprüche 1 bis 7, **dadurch gekennzeichnet, daß** die Umsetzung bei Drücken von 0,1 bis 20 MPa, bevorzugt 1 bis 12 MPa und insbesondere 3 bis 7 MPa erfolgt.

9. In organischer aldehydhaltiger Phase nicht löslicher Katalysator zur Hydroformylierung von Monoolefinen, nichtkonjugierten Polyolefinen, Cycloolefinen oder Derivaten dieser Verbindungsklasse mit 2 bis 20 Kohlenstoffatomen enthaltend Rhodium und eine nichtwäßrige ionogenen Ligandflüssigkeit der allgemeinen Formel (Q^{⊕})ₐ A^{a-}, **dadurch gekennzeichnet, daß** Q^{⊕} für quaternäre Ammonium- und/oder Phosphonium-Kationen der allgemeinen Formeln 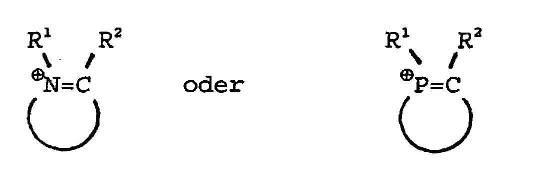 steht, die 1, 2 oder 3 Stickstoffatome und/oder Phosphoratome im Ring tragen, wobei R¹,R² gleich oder verschieden sind und Wasserstoff oder einen geradkettigen oder verzweigten Kohlenwasserstoffrest mit 1 bis 20 Kohlenstoffatomen bedeuten und in denen die Heterocyclen aus 4 bis 10 Ringatomen bestehen; oder dass Q^{⊕} für quaternäre Ammonium- und/oder Phosphonium-Kationen der allgemeinen Formeln R¹R^{2⊕}N=CR³-X-R³C=^{⊕}NR¹R² oder R¹R^{2⊕}P=CR³-X-R³C=^{⊕}PR¹R² steht, wobei R¹,R² und R³ gleich oder verschieden sind und Wasserstoff oder einen geradkettigen oder verzweigten Kohlenwasserstoffrest mit 1 bis 20 Kohlenstoffatomen und X einen Alkylen- oder Phenylenrest bedeutet; oder dass Q^{⊕} für das N-Butylpyridinium-, N-Ethylpyridinium-, 1-n-Butyl-3-methylimidazolium-, Diethylpyrazolium-, 1-Ethyl-3-methylimidazolium-, Pyridinium-Kation, für das Tricyclodecan Diammonium-Kation oder das N,N'-Dimethyl-Tricyclodecan Diammonium-Kation steht; oder dass Q^{⊕} für quaternäre Ammonium- und/oder Phosphonium-Kationen der allgemeinen Formeln R¹R²R³N^{⊕} (̵ X )̵ N^{⊕}R⁴R⁵R⁶ oder R¹R²R³P^{⊕} (̵ X )̵ P^{⊕}R⁴R⁵R⁶ steht, in der R¹, R², R³, R⁴, R⁵ und R⁶ gleich oder verschieden sind und Wasserstoff oder einen geradkettigen oder verzweigten Kohlenwasserstoffrest mit 1 bis 20 Kohlenstoffatomen bedeuten, und X für 1,2-Phenylen, 1,3-Phenylen, 1,4-Phenylen oder für einen Alkylenrest (̵CHR⁷)̵_{b}, wobei R⁷ gleich Wasserstoff oder ein Kohlenwasserstoffrest mit 1 bis 5 Kohlenstoffatomen ist, und b eine ganze Zahl zwischen 1 bis 8 ist, steht und A^{a-} ein Triarylphosphin der allgemeinen Formel 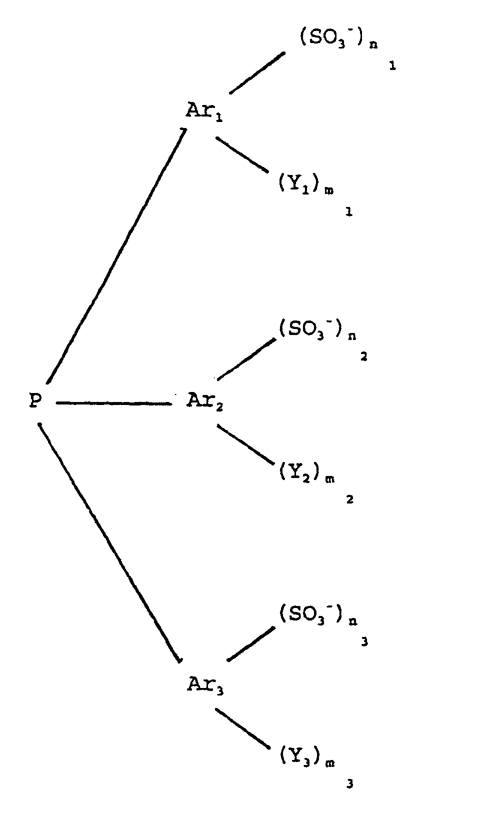 ist, in der Ar¹, Ar² und Ar³ gleiche oder verschiedene Arylgruppen mit 6 bis 14 Kohlenstoffatomen bedeuten,die Substituenten Y₁, Y₂ und Y₃ gleiche oder verschiedene geradkettige oder verzweigte Alkyloder Alkoxyreste mit 1 bis 4 Kohlenstoffatomen bedeuten, Chlor, Brom, die Hydroxyl-, Cyanid- oder Nitrogruppe bedeuten, ferner für die Aminogruppe der Formel NR⁸R⁹ stehen, in der die Substituenten R⁸ und R⁹ gleich oder verschieden sind und Wasserstoff, geradkettige oder verzweigte Alkylgruppen mit 1 bis 4 Kohlenstoffatomen bedeuten, in der m₁, m₂ und m₃ gleich oder verschieden sind und ganze Zahlen von 0 bis 5 bedeuten; in der n₁, n₂ und n₃ gleich oder verschieden sind und für ganze Zahlen von 0 bis 3 stehen, wobei mindestens eine der Zahlen n₁, n₂ und n₃ gleich oder größer 1 ist, und in der a gleich n₁+n₂+n₃ ist, und daß über die stöchiometrisch erforderliche Menge zur Bildung von (Q^{⊕})ₐ A^{a-} hinaus ein Überschuß an aus Q^{⊕} abgeleiteten Aminen und/oder Phosphinen von bis zu 5 Äquivalenten oder ein Überschuß an Alkali- und/oder Erdalkalisalzen der Triarylphosphine A^{a-} von bis zu 5 Äquivalenten vorliegt, und dass je mol Rhodium 2 bis 1000 mol Phosphor(III) vorliegen.

## Claims

1. A two-phase process for preparing aldehydes by reacting monoolefins, non-conjugated polyolefins, cycloolefins or derivatives of this class of compounds having from 2 to 20 carbon atoms with carbon monoxide and hydrogen at temperatures of from 20 to 150°C and pressures of from 0.1 to 20 MPa, **characterized in** the presence of an organic aldehyde containing phase and a non-aqueous ionic ligand liquid of the formula (Q^{⊕})ₐA^{a-}, non-soluble therein, with at least one rhodium compound, wherein Q^{⊕} is a quaternary ammonium and/or phosphonium cation of the formula 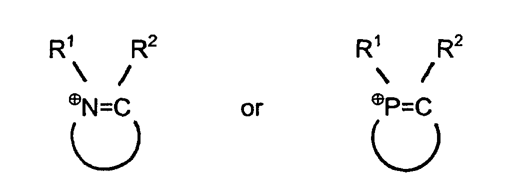 which have 1, 2 or 3 nitrogen atoms and/or phosphorus atoms in the ring, where R¹, R² are identical or different and are each hydrogen or a straight-chain or branched hydrocarbon radical having from 1 to 20 carbon atoms and in which the heterocycles have from 4 to 10 ring atoms; or wherein Q^{⊕} is a quaternary ammonium and/or phosphonium cation of the formula R¹R^{2⊕}N=CR³-X-R³C=^{⊕}NR¹R² or R¹R^{2⊕}P=CR³-X-R³C=^{⊕}PR¹R², where R¹, R² and R³ are identical or different and are each hydrogen or a straight-chain or branched hydrocarbon radical having from 1 to 20 carbon atoms and X is an alkylene or phenylene radical; or wherein Q^{⊕} is the N-butylpyridinium, N-ethylpyridinium, 1-n-butyl-3-methylimidazolium, diethylpyrazolium, 1-ethyl-3-methylimidazolium, pyridinium cation, the tricyclodecanediammonium cation or the N,N'-dimethyltricyclodecane-diammonium cation; or wherein Q^{⊕} is a quaternary ammonium and/or phosphonium cation of the formula R¹R²R³N^{⊕} (̵X)̵ N^{⊕}R⁴R⁵R⁶ or R¹R²R³P^{⊕} (̵X)̵ P^{⊕}R⁴R⁵R⁶ in which R¹, R², R³, R⁴, R⁵ and R⁶ are identical or different and are each hydrogen or a straight-chain or branched hydrocarbon radical having from 1 to 20 carbon atoms and X is 1,2-phenylene, 1,3-phenylene, 1 ,4-phenylene or an alkylene radical (̵ CHR⁷)̵_{b}, where R⁷ is hydrogen or a hydrocarbon radical having from 1 to 5 carbon atoms and b is an integer from 1 to 8, and A^{a-} is a triarylphosphine of the formula 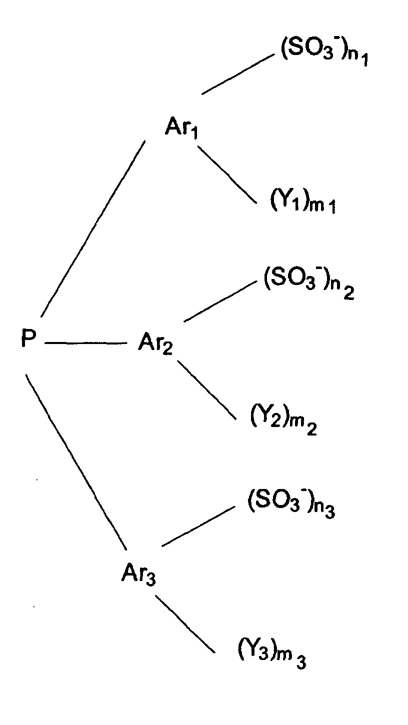 where Ar¹, Ar² and Ar³ are identical or different aryl groups having from 6 to 14 carbon atoms, the substituents Y₁, Y₂ and Y₃ are identical or different straight-chain or branched alkyl or alkoxy radicals having from 1 to 4 carbon atoms, chlorine, bromine, hydroxyl, cyano, nitro or amino groups of the formula NR⁸R⁹, where the substituents R⁸ and R⁹ are identical or different and are hydrogen, straight-chain or branched alkyl groups having from 1 to 4 carbon atoms,
m₁, m₂ and m₃ are identical or different and are integers from 0 to 5,
n₁, n₂ and n₃ are identical or different and are integers from 0 to 3, where at least one of the numbers n₁, n₂ and n₃ is equal to or greater than 1, and
a is n₁+n₂+n₃,
and wherein amines and/or phosphines derived from Q^{⊕} are present in an excess of up to 5 equivalents over the stoichiometrically required amounts for the formation of (Q^{⊕})ₐA^{a-} or alkali metal and/or alkaline earth metal salts of the triarylphosphine A^{a-} are present in an excess of up to 5 equivalents over the stoichiometrically required amounts for the formation of (Q^{⊕})ₐA^{a-} and wherein 2 to 1000 mol of phosphorus(III) are used per mol of rhodium and wherein the organic aldehyd-containing phase and the rhodium-containing nonaqueous ionic ligand liquid are separated from one another by phase separation and the rhodium-containing nonaqueous ionic ligand liquid which has been separated off is returned completely or partially to the hydroformylation reactor.

2. The process as claimed in claim 1, wherein amines and/or phosphines derived from (Q^{⊕}) are present in an excess of up to 1 equivalent over the stoichiometrically required amount for the formation of (Q^{⊕})ₐA^{a-} or alkali metal or alkaline earth metal salts of the triarylphosphine A^{a-} are present in an excess of up to 1 equivalent over the stoichiometrically required amount for the formation of (Q^{⊕})ₐA^{a-}.

3. The process as claimed in claim 1 or 2, wherein R¹, R², R³, R⁴, R⁵ and R⁶ are identical or different and are hydrogen, n-butyl, n-pentyl, n-hexyl, n-heptyl, i-heptyl, n-octy, i-octyl, n-nonyl, i-nonyl, n-decyl, i-decyl, n-undecyl, i-undecyl, n-dodecyl or i-dodecyl, R⁷ is hydrogen, methyl or ethyl and b is 2, 3, 4, 5 or 6.

4. The process as claimed in claim 3, wherein R¹ and R² are identical or different and are n-butyl, n-pentyl, n-hexyl, n-heptyl, i-heptyl, n-octyl, i-octyl, n-nonyl, i-nonyl, n-decyl, i-decyl, n-undecyl, i-undecyl, n-dodecyl or i-dodecyl, R³, R⁴, R⁵ and R⁶ are hydrogen, R⁷ is hydrogen and b is 3.

5. The process as claimed in one or more of claims 1 to 4, wherein 3 to 300 mol, in particular from 20 to 100 mol, of phosphorus(III) are used per mole of rhodium.

6. The process as claimed in any of claims 1 to 5, wherein the rhodium concentration is from 2 to 1000 ppm by weight, preferably from 3 to 400 ppm by weight and in particular from 5 to 100 ppm by weight, based on the amount of olefin used.

7. The process as claimed in any of claims 1 to 6, wherein the reaction is carried out at from 20 to 150°C, preferably from 80 to 140°C and in particular from 100 to 125°C.

8. The process as claimed in any of claims 1 to 7, wherein the reaction is carried out at pressures of from 0.1 to 20 MPa, preferably from 1 to 12 MPa and in particular from 3 to 7 MPa.

9. In an organic aldehyde containing phase non soluble catalyst for the hydroformylation of monoolefins, non-conjugated polyolefins, cycloolefins or derivatives of this class of compounds having from 2 to 20 carbon atoms comprising rhodium and a nonaqueous ionic ligand liquid of the formula (Q^{⊕})ₐA^{a-}, wherein Q^{⊕} is a quaternary ammonium and/or phosphonium cation of the formula 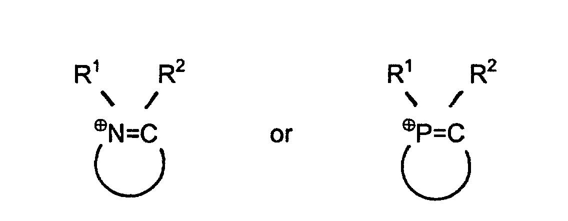 which have 1, 2 or 3 nitrogen atoms and/or phosphorus atoms in the ring, where R¹, R² are identical or different and are each hydrogen or a straight-chain or branched hydrocarbon radical having from 1 to 20 carbon atoms and in which the heterocycles have from 4 to 10 ring atoms; or wherein Q^{⊕} is a quatemary ammonium and/or phosphonium cation of the formula R¹R^{2⊕}N=CR³-X-R³C=^{⊕}NR¹R² or R¹R^{2⊕}P=CR³-X-R³C=^{⊕}PR¹R², where R¹, R² and R³ are identical or different and are each hydrogen or a straight-chain or branched hydrocarbon radical having from 1 to 20 carbon atoms and X is an alkylene or phenylene radical, or wherein Q^{⊕} is the N-butylpyridinium, N-ethylpyridinium, 1-n-butyl-3-methylimidazolium, diethylpyrazolium, 1-ethyl-3-methylimidazolium, pyridinium cation, the tricyclodecanediammonium cation or the N,N'-dimethyltricyclodecane-diammonium cation; or wherein Q^{⊕} is a quaternary ammonium and/or phosphonium cation of the formula R¹R²R³N^{⊕} (̵ X )̵ N^{⊕}R⁴R⁵R⁶ or R¹R²R³P^{⊕} (̵X)̵ P^{⊕}R⁴R⁵R⁶ in which R¹, R², R³, R⁴, R⁵ and R⁶ are identical or different and are each hydrogen or a straight-chain or branched hydrocarbon radical having from 1 to 20 carbon atoms and X is 1,2-phenylene, 1,3-phenylene, 1,4-phenylene or an alkylene radical (̵ CHR⁷ )̵ _{b}, where R⁷ is hydrogen or a hydrocarbon radical having from 1 to 5 carbon atoms and b is an integer from 1 to 8; and A^{a-} is a triarylphosphine of the formula 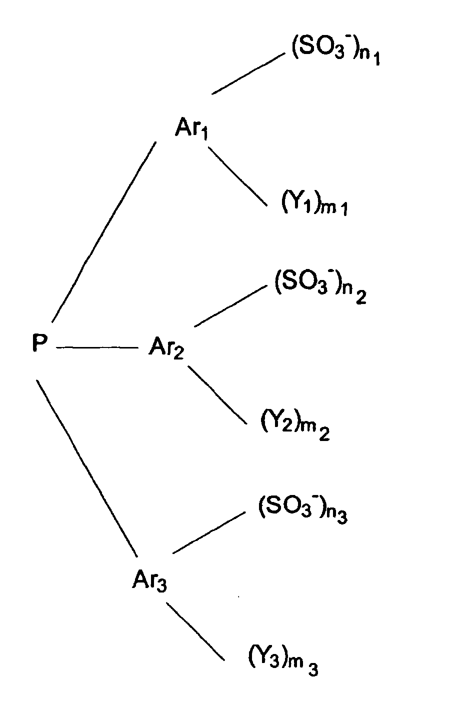 where Ar¹, Ar² and Ar³ are identical or different aryl groups having from 6 to 14 carbon atoms, the substituents Y₁, Y₂ and Y₃ are identical or different straight-chain or branched alkyl or alkoxy radicals having from 1 to 4 carbon atoms, chlorine, bromine, hydroxyl, cyano, nitro or amino groups of the formula NR⁸R⁹, where the substituents R⁸ and R⁹ are identical or different and are hydrogen, straight-chain or branched alkyl groups having from 1 to 4 carbon atoms,
m₁, m₂ and m₃ are identical or different and are integers from 0 to 5, n₁, n₂ and n₃ are identical or different and are integers from 0 to 3, where at least one of the numbers n₁, n₂ and n₃ is equal to or greater than 1, and
a is n₁+n₂+n₃,
and wherein amines and/or phosphines derived from Q^{⊕} are present in an excess of up to 5 equivalents over the stoichiometrically required amounts for the formation of (Q^{⊕})ₐA^{a-} or alkali metal and/or alkaline earth metal salts of the triarylphosphine A^{a-} are present in an excess of up to 5 equivalents over the stoichiometrically required amounts for the formation of (Q^{⊕})ₐA^{a-}, and wherein 2 to 1000 mol of phosphorus(III) are used per mol of rhodium.

## Revendications

1. Procédé dans un milieu à deux phases pour la préparation d'aldéhydes par réaction de mono-oléfines, de polyoléfines non conjuguées, de cyclo-oléfines ou de dérivés en C₂ à C₂₀ de cette classe de composés avec le monoxyde de carbone et l'hydrogène à des températures de 20 à 150°C et des pressions de 0,1 à 20 MPa, **caractérisé en ce que** l'on opère en présence d'une phase organique contenant l'aldéhyde et d'un liquide ligand ionogène non aqueux non soluble dans la phase organique, répondant à la formule générale (Q^{⊕})ₐA^{a-} avec au moins un dérivé du rhodium, **caractérisé en ce que** Q^{⊕} représente un cation ammonium quaternaire et/ou phosphonium quaternaire répondant à l'une des formules générales respectives 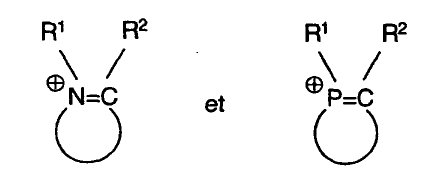 dans lesquelles le cycle contient 1, 2 ou 3 atomes d'azote et/ou de phosphore, R¹ et R², ayant des significations identiques ou différentes, représentent chacun l'hydrogène ou un radical hydrocarboné à chaîne droite ou ramifiée en C₁ à C₂₀, et les hétérocycles contiennent 4 à 10 atomes cycliques ;
ou bien Q^{⊕} représente un cation ammonium quaternaire et/ou phosphonium quaternaire répondant à l'une des formules générales respectives R¹R^{2⊕}N=CR³-X-R³C=^{⊕}NR¹R² et R¹R^{2⊕}P=CR³-X-R³C=^{⊕}PR¹R² dans lesquelles R¹, R² et R³ ayant des significations identiques ou différentes représentent chacun l'hydrogène ou un radical hydrocarboné à chaîne droite ou ramifiée en C₁ à C₂₀ et X représente un groupe alkylène ou phénylène ;
ou bien Q^{⊕} représente un cation N-butylpyridinium, N-éthylpyridinium, 1-n-butyl-3-méthylimidazolium, diéthylpyrazolium, 1-éthyl-3-méthylimidazolium, pyridinium, un cation tricyclodécane-diammonium ou un cation N,N'-diméthyltricyclodécanediammonium ;
ou bien Q^{⊕} représente un cation ammonium quaternaire et/ou phosphonium quaternaire répondant à l'une des formules générales respectives R¹R²R³N^{⊕} (̵ X )̵ N^{⊕}R⁴R⁵R⁶ et R¹R²R³P^{⊕} (̵ X )̵ P^{⊕}R⁴R⁵R⁶, et dans lesquelles R¹, R², R³, R⁴, R⁵ et R⁶, ayant des significations identiques ou différentes, représentent chacun l'hydrogène ou un radical hydrocarboné à chaîne droite ou ramifiée en C₁ à C₂₀, et X représente un groupe 1,2-phénylène, 1,3-phénylène, 1,4-phénylène, ou un groupe alkylène (̵CHR⁷)̵_{b}, dans lequel R⁷ représente l'hydrogène ou un radical hydrocarboné en C₁ à C₅, et b est un nombre entier allant de 1 à 8, et A^{a-} est une triarylphosphine de formule générale 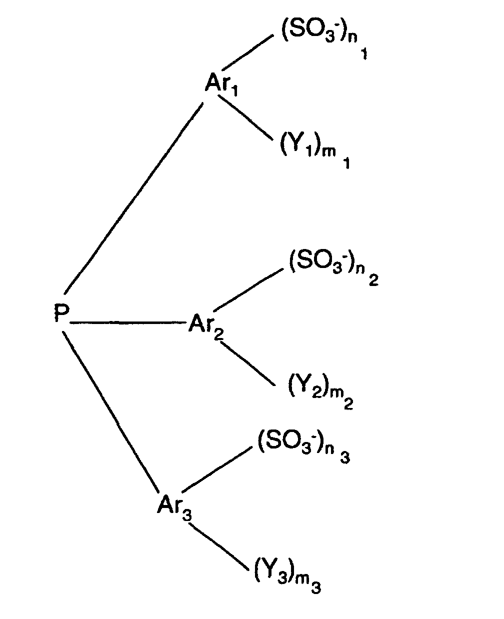 dans laquelle Ar¹, Ar² et Ar³ ayant des significations identiques ou différentes représentent chacun un groupe aryle en C₆ à C₁₄, Y₁, Y₂ et Y₃, ayant des significations identiques ou différentes représentent chacun un groupe alkyle ou alcoxy à chaîne droite ou ramifiée en C₁ à C₄, le chlore, le brome, un groupe hydroxy, cyanure ou nitro, ou encore le groupe amino de formule NR⁸R⁹ dans lequel R⁸ et R⁹, ayant des significations identiques ou différentes, représentent chacun l'hydrogène, un groupe alkyle à chaîne droite ou ramifiée en C₁ à C₄, m₁, m₂ et m₃ sont des nombres entiers égaux ou différents allant de 0 à 5 ; n₁, n₂ et n₃ sont des nombres entiers égaux ou différents allant de 0 à 3, l'un des nombres n₁, n₂ et n₃ étant égal ou supérieur à 1, a est égal à n₁+n₂+n₃, et **en ce que**, en plus de la quantité stoechiométrique nécessaire pour la formation de (Q^{⊕})ₐA^{a-}, il y a un excès d'amines et/ou de phosphines dérivées de Q^{⊕} allant jusqu'à 5 équivalents ou un excès de sels alcalins et/ou alcalino-terreux des triphénylphosphines A^{a-} allant jusqu'à 5 équivalents, et **en ce que** l'on utilise 2 à 1000 mol de phosphore (III) par mole de rhodium, et **en ce que** l'on sépare la phase organique contenant l'aldéhyde et le liquide ligand ionogène non aqueux contenant le rhodium par décantation, et on réintroduit le liquide ligand ionogène non aqueux contenant le rhodium séparé en totalité ou en partie dans le réacteur d'hydroformylation.

2. Procédé selon la revendication 1, **caractérisé en ce que**, en plus de la quantité stoechiométrique nécessaire pour la formation de (Q^{⊕})ₐA^{a-}, il y a un excès d'amines et/ou de phosphines dérivées de Q^{⊕} allant jusqu'à un équivalent ou un excès de sels alcalins ou alcalino-terreux des triarylphosphines A^{a-} allant jusqu'à un équivalent.

3. Procédé selon la revendication 1 ou 2, **caractérisé en ce que** R¹, R², R³, R⁴, R⁵ et R⁶ ayant des significations identiques ou différentes représentent chacun l'hydrogène, un groupe n-butyle, n-pentyle, n-hexyle, n-heptyle, iso-heptyle, n-octyle, iso-octyle, n-nonyle, iso-nonyle, n-décyle, iso-décyle, n-undécyle, iso-undécyle, n-dodécyle ou iso-dodécyle, R⁷ représente l'hydrogène, un groupe méthyle ou éthyle et b est égal à 2, 3, 4, 5 ou 6.

4. Procédé selon la revendication 3, **caractérisé en ce que** R¹ et R², ayant des significations identiques ou différentes, représentent chacun un groupe n-butyle, n-pentyle, n-hexyle, n-heptyle, iso-heptyle, n-octyle, iso-octyle, n-nonyle, iso-nonyle, n-décyle, iso-décyle, n-undécyle, iso-undécyle, n-dodécyle ou iso-dodécyle, R³, R⁴, R⁵ et R⁶ représentent l'hydrogène, R⁷ représente l'hydrogène et b est égal à 3.

5. Procédé selon une ou plusieurs des revendications 1 à 4, **caractérisé en ce que** l'on utilise 3 à 300, plus spécialement 20 à 100 mol de phosphore (III) par mole de rhodium.

6. Procédé selon l'une des revendications 1 à 5, **caractérisé en ce que** la concentration en rhodium représente de 2 à 1000 ppm en poids, de préférence de 3 à 400 ppm en poids, et plus spécialement de 5 à 100 ppm en poids par rapport à la quantité d'oléfine mise en oeuvre.

7. Procédé selon l'une des revendications 1 à 6, **caractérisé en ce que** la réaction est réalisée à des températures de 20 à 150°C, de préférence de 80 à 140°C et plus spécialement de 100 à 125°C.

8. Procédé selon l'une des revendications 1 à 7, **caractérisé en ce que** la réaction est réalisée sous des pressions de 0,1 à 20 MPa, de préférence de 1 à 12 MPa et plus spécialement de 3 à 7 MPa.

9. Catalyseur pour l'hydroformylation de mono-oléfines, de polyoléfines non conjuguées, de cyclo-oléfines ou de dérivés de cette classe de composés en C₂ C₂₀, non soluble dans la phase organique contenant l'aldéhyde, contenant du rhodium et un liquide ligand ionogène non aqueux de formule générale (Q^{⊕})ₐA^{a-}, **caractérisé en ce que** Q^{⊕} représente un cation ammonium quaternaire et/ou phosphonium quaternaire répondant à l'une des formules générales respectives 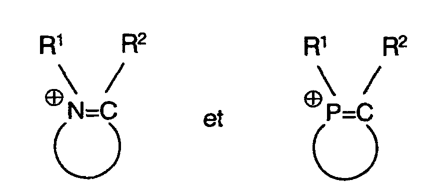 dans lesquelles les cycles contiennent 1, 2 ou 3 atomes d'azote et/ou de phosphore, R¹ et R², ayant des significations identiques ou différentes, représentent chacun l'hydrogène ou un radical hydrocarboné à chaîne droite ou ramifiée en C₁ à C₂₀, et les hétérocycles consistent en 4 à 10 atomes cycliques ;
ou bien Q^{⊕} représente un cation ammonium quaternaire et/ou phosphonium quaternaire répondant à l'une des formules générales respectives R¹R^{2⊕}N=CR³-X-R³C=^{⊕}NR¹R² et R¹R^{2⊕}P=CR³-X-R³C=^{⊕}PR¹R² dans lesquelles R¹, R² et R³ ayant des significations identiques ou différentes représentent chacun l'hydrogène ou un radical hydrocarboné à chaîne droite ou ramifiée en C₁ à C₂₀ et X représente un groupe alkylène ou phénylène ;
ou bien Q^{⊕} représente un cation N-butylpyridinium, N-éthylpyridinium, 1-n-butyl-3-méthylimidazolium, diéthylpyrazolium, 1-éthyl-3-méthylimidazolium, pyridinium, un cation tricyclodécane-diammonium ou un cation N,N'-diméthyltricyclodécanediammonium ;
ou bien Q^{⊕} représente un cation ammonium quaternaire et/ou phosphonium quaternaire répondant à l'une des formules générales respectives R¹R²R³N^{⊕} (̵X)̵ N^{⊕}R⁴R⁵R⁶ et R¹R²R³P^{⊕} (̵X)̵ P^{⊕}R⁴R⁵R⁶, dans lesquelles R¹, R², R³, R⁴, R⁵ et R⁶ ayant des significations identiques ou différentes, représentent chacun l'hydrogène ou un radical hydrocarboné à chaîne droite ou ramifiée en C₁ à C₂₀, et X représente un groupe 1,2-phénylène, 1,3-phénylène, 1,4-phénylène, ou un groupe alkylène (̵CHR⁷)̵_{b} dans lequel R⁷ représente l'hydrogène ou un radical hydrocarboné en C₁ à C₅, et b est un nombre entier allant de 1 à 8, et A^{a-} est une triarylphosphine de formule générale 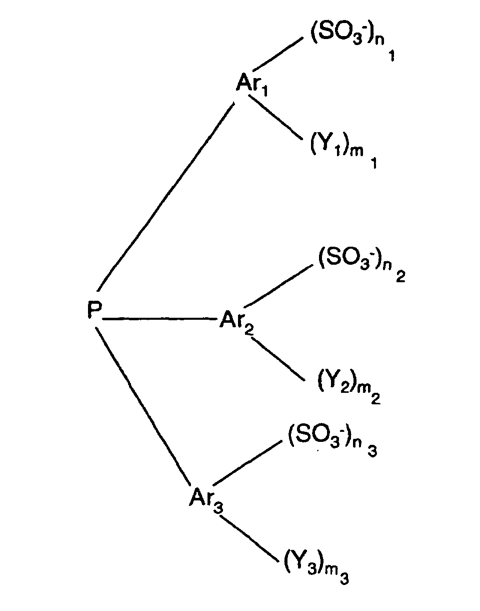 dans laquelle Ar¹, Ar² et Ar³ ayant des significations identiques ou différentes représentent chacun un groupe aryle en C₆ à C₁₄, Y₁, Y₂ et Y₃ ayant des significations identiques ou différentes représentent chacun un groupe alkyle ou alcoxy à chaîne droite ou ramifiée en C₁ à C₄, le chlore, le brome, un groupe hydroxy, cyanure ou nitro, ou encore un groupe amino de formule NR⁸R⁹ dans laquelle R⁸ et R⁹, ayant des significations identiques ou différentes, représentent chacun l'hydrogène, un groupe alkyle à chaîne droite ou ramifiée en C₁ à C₄, m₁, m₂ et m₃ sont des nombres entiers égaux ou différents allant de 0 à 5 ; n₁, n₂ et n₃ sont des nombres entiers égaux ou différents allant de 0 à 3, l'un au moins des nombres n₁, n₂ et n₃ étant égal ou supérieur à 1, et a est égal à n₁+n₂+n₃, et **en ce que**, en plus de la quantité stoechiométrique nécessaire pour la formation de (Q^{⊕})ₐA^{a-}, il y un excès d'amines et/ou de phosphines dérivées de Q^{⊕} allant jusqu'à 5 équivalents ou un excès de sels alcalins et/ou alcalino-terreux des triarylphosphines A^{a-} allant jusqu'à 5 équivalents, et **en ce qu'**il y a de 2 à 1000 mol de phosphore (III) par mole de rhodium.
